(19) **Europäisches Patentamt**
**European Patent Office**
**Office européen des brevets**

(11) **EP 2 529 659 A1**

(12) **EUROPEAN PATENT APPLICATION**

(43) Date of publication:
**05.12.2012 Bulletin 2012/49**

(51) Int Cl.:
***A61B 1/06*** (2006.01)

(21) Application number: **12170060.3**

(22) Date of filing: **30.05.2012**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **31.05.2011 JP 2011121199**

(71) Applicant: **Fujifilm Corporation**
**Minato-ku**
**Tokyo (JP)**

(72) Inventors:
- **Kasamatsu, Tadashi**
  **Kanagawa-ken (JP)**
- **Yoshihiro, Tatsuya**
  **Kanagawa-ken (JP)**
- **Yoshida, Koji**
  **Kanagawa-ken (JP)**
- **Ohashi, Eiji**
  **Kanagawa-ken (JP)**

(74) Representative: **Klunker . Schmitt-Nilson . Hirsch**
**Patentanwälte**
**Destouchesstrasse 68**
**80796 München (DE)**

(54) **Light source apparatus**

(57)     Setting ratio between projection range of excitation light and projection range of white light to a desired predetermined value while maintaining sufficient biological surface illuminance for both the white light and excitation light. A light source apparatus includes a first light source (52) for emitting first light (e.g., excitation light) which is inputted to a light guide section (LG) that guides light to an examination area and projects the light onto the examination area and a second light source (50) for emitting second light (e.g., white light) which is inputted to the light guide (LG) in which exit angles of the first and second light are changed simultaneously by an exit angle changing section (57), the first and second light being guided by the light guide section (LG) and projected onto the examination area.

**EP 2 529 659 A1**

**Description**

BACKGROUND OF THE INVENTION

Field of the Invention

The present invention relates to a light source apparatus for inputting light having a first wavelength range and light having a second wavelength range different from the first wavelength range to a light incident end face of a predetermined light guide section.

Description of the Related Art

Endoscope systems for observing tissues of body cavities are widely known, and electronic endoscope systems for capturing an ordinary image of an examination area in a body cavity by projecting white light onto the examination area and displaying the captured ordinary image on a monitor screen are widely used.

Further, as one of such endoscope systems, a system in which ICG (indocyanine green) is administered to an examination area in advance in order to examine, for example, a blood vessel run, blood flow, lymphatic vessel, lymph flow, bile duct run, or bile flow under fat which does not appear in an ordinary image and an ICG florescence image is captured by projecting near infrared excitation light to the examination area is proposed. Another type of endoscope system that obtains a fluorescence image by projecting excitation light to an examination area and detecting autofluorescence emitted from the examination area is also proposed.

An endoscope system that captures a fluorescence image by projecting excitation light to an examination area as described above, as well as an ordinary image by projecting white light to the examination area, is proposed as described, for example, U.S. Patent Application Publication No. 20020026099.

In the endoscope system described in U.S. Patent Application Publication No. 20020026099, it is proposed that the projection range of excitation light on an examination area is made to agree with the projection range of white light so that, when generating a diagnostic image by superimposing a fluorescence image and an ordinary image on top of each other, these images may correspond to each other.

More specifically, it is proposed that the incident angle of excitation light on a light incident end face of a light guide for guiding the excitation light is changed by changing the focal length of a lens to which only the excitation light is inputted, thereby making the projection range of the excitation light to agree with the fixed projection range of the white light.

Incidentally, in surgical treatment sites, for example, endoscope systems are generally used for observing proximal sites, such as stomach cancer and the like. But, there may be a case in which a distal site, such as peritoneal seeding and the like, needs to be examined.

In such a case, use of the endoscope system described in U.S. Patent Application Publication No. 20020026099 may cause a problem that the illuminance of white light at the distal side may be reduced as the exit angle of the white light is fixed, although the illuminance of the excitation light may be maintained at a sufficient level at the distal site by narrowing the exit angle thereof.

Further, even if the exit angle of the white light is made changeable in the endoscope system described in U.S. Patent Application Publication No. 20020026099, a problem that an image with an inappropriate ratio between the projection range (radius) of excitation light and projection range (radius) of white light is captured or otherwise a complicated procedure for adjusting the ratio is required if there is not any consistent relationship between the change in exit angle of the excitation light and change in exit angle of the white light. More specifically, when non-visible light, such as near infrared right, is used as the excitation light and if the ratio between the projection range of excitation light and projection range of white light is not maintained consistent, the user can not see which region the excitation light is projected with respect to the white light projection region. Consequently, there may be a case in which a determination can not be made as to whether there is not any lesion and no fluorescence image is captured or there exists a lesion but a fluorescence image thereof is not captured because the lesion is present outside of the projection range of the excitation light and, therefore, a care must be taken not to make a false-negative judgment in the image diagnosis. There may also be a case in which a determination can not be made as to whether there is a lesion and a fluorescence image thereof is captured or there exists no lesion but a fluorescence image of a normal tissue is captured as a lesion, which should not basically occur, because the projection range of the excitation light is too narrow and illuminance thereof is too high and, therefore, a care must be taken not to make a false-positive judgment in the image diagnosis.

In view of the circumstances described above, it is an object of the present invention to provide a light source apparatus capable of appropriately adjusting the ratio between the projection range of excitation light and projection range of white light, while maintaining sufficient biological surface illuminance for both the white light and excitation light.

## SUMMARY OF THE INVENTION

A light source apparatus of the present invention is an apparatus, including:

a first light source for emitting first light having a first wavelength range and is inputted to a light guide section that guides light to an examination area and projects the light onto the examination area;
a second light source for emitting a second light having a second wavelength range different from the first wavelength range and is inputted to the light guide section; and
an exit angle changing section for simultaneously changing exit angles of the first and second light from the light guide section, the fist and second light being guided by the light guide section and projected onto the examination area.

In the light source apparatus of the present invention, the exit angle changing section may include an incident angle changing optical system for changing the exit angles by changing incident angles of the first and second light on a light incident end face of the light guide section.

Further, the exit angle changing section may change the incident angles by changing a focal length of the incident angle changing optical system.

Still further, the exit angle changing section may include an upstream optical system for receiving either one of the first and second light and inputting the received either one of the light to the incident angle changing optical system; and

a ratio between the exit angles of the first and second light from the light guide section is maintained constant by changing an incident height of the either one of the light to be inputted to the incident angle changing optical system by the upstream optical system along with the change in the exit angles by the incident angle changing optical system.

Further, the second light emitted from the second light source may be guided parallel to optical axis of the incident angle changing optical system while the first light emitted from the first light source maybe guided perpendicular to the optical axis of the incident angle changing optical system, and the apparatus may include a mirror for combining the parallel-guided second light and the perpendicular-guided first light.

Still further, the first light emitted from the first light source may be guided parallel to an optical axis of the incident angle changing optical system while the second light emitted from the second light source may be guided perpendicular to the optical axis of the incident angle changing optical system, and the apparatus may include a mirror for combining the parallel-guided first light and the perpendicular-guided second light.

Further, a zoom lens may be used as the incident angle changing optical system.

Still further, a zoom beam expander maybe used as the upstream optical system.

Further, the exit angle changing section may include a first light exit angle changing section for changing the exit angle of the first light and a second light exit angle changing section for changing the exit angle of the second light.

Still further, the first light exit angle changing section may change the exit angle of the first light by changing an incident angle of optical axis of the first light with respect to optical axis of the light guide section.

Further, a semiconductor light source may be used as the first light source.

Still further, the first light source may emit near infrared light as the first light while the second light source may be a visible light source.

According to the light source apparatus of the present invention, exit angles of first and second light guided by the light guide section and projected onto an examination area are changed simultaneously. This allows the ratio between the projection range of the first light and projection range of the second light to be set to a desired predetermined value without requiring complicated operations, while maintaining sufficient biological surface illuminance for both the first and second light.

Thus, in the case where the first light is near infrared excitation light and the second light is visible light, the ratio between the projection range of the excitation light and the projection range of the visible light may be maintained constant, and the user may know the ratio, so that the user may know to which portion the excitation light is projected with respect to the projection range of the visible light. Consequently, false-negative and false positive judgments may be avoided.

Further, in the case where the upstream optical system for receiving either one of the first and second light and inputting the received either one of the light to the incident angle changing optical system is further provided, and incident height of the either one of the light to be inputted to the incident angle changing optical system by the upstream optical system is changed along with the change in the exit angles by the incident angle changing optical system, another advantageous effect may be obtained. That is, it is more effective to the feeling of the user to maintain the ratio between exit angles of the first and second light constant rather than maintaining the ratio between the radii of projection ranges thereof constant.

BRIEF DESCRIPTION OF THE DRAWINGS

Figure 1 is a schematic configuration diagram of a rigid endoscope system that employs an embodiment of a light source apparatus of the present invention.

Figure 2 is a schematic configuration diagram of the body cavity insertion section shown in Figure 1.

Figure 3 is a schematic configuration diagram of an imaging unit.

Figure 4 illustrates a schematic configuration of an image processing unit and a light source apparatus (prior to change in exit angle) according to a first embodiment.

Figure 5 illustrates a schematic configuration of the image processing unit and the light source apparatus (after change in exit angle) according to the first embodiment.

Figure 6 is a graph illustrating an example relationship between incident angles of excitation light and white light.

Figure 7 illustrates a schematic configuration of the image processing unit and a light source apparatus (prior to change in exit angle) according to a second embodiment.

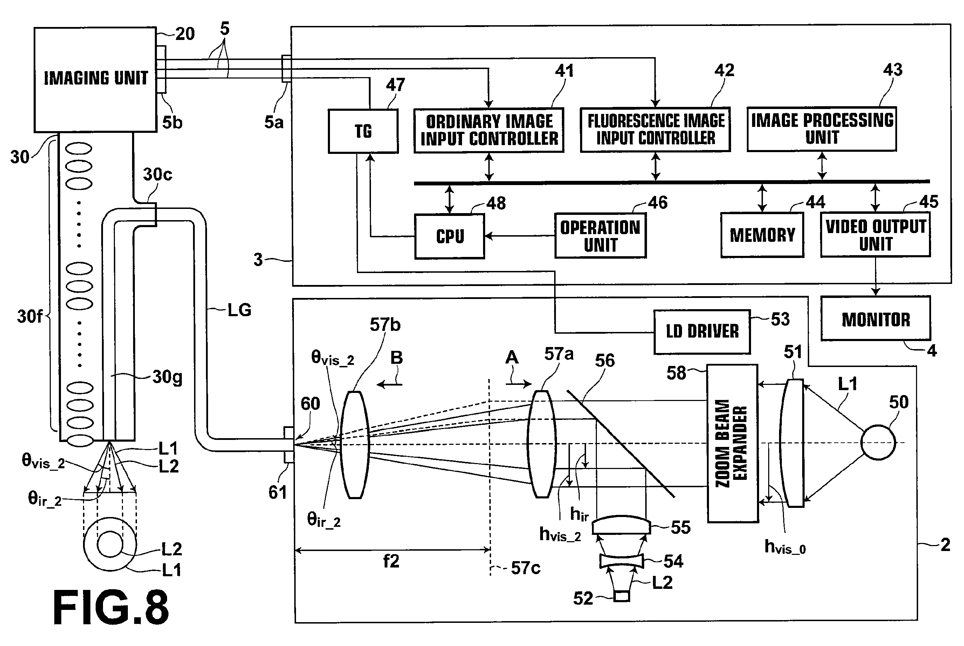

Figure 8 illustrates a schematic configuration of the image processing unit and the light source apparatus (after change in exit angle) according to the second embodiment.

Figure 9 is a graph illustrating an example relationship between the incident angle and incident height of white light.

Figure 10 is a graph illustrating an example relationship between the focal length of a zoom lens and magnification ratio of a zoom expander.

Figure 11 illustrates an alternative embodiment (high incident height) of the zoom expander.

Figure 12 illustrates an alternative embodiment (low incident height) of the zoom expander.

Figure 13 is a graph illustrating an example relationship between the focal length of a zoom lens and the focal length of a zoom lens in the zoom expander.

Figure 14 is a schematic configuration diagram of a light source apparatus according to a third embodiment of the present invention.

Figure 15 is a schematic configuration diagram of a light source apparatus according to a fourth embodiment of the present invention.

Figure 16 is a graph illustrating experimental data of an illuminance distribution when excitation light is incident on the light incident end face of the light guide from a direction inclined by an incident angle of 20° with respect to a direction perpendicular to the light incident end face and of an illuminance distribution when the excitation light is incident on the light incident end face of the light guide from a direction perpendicular to the light incident end face of the light guide.

Figure 17 is a schematic configuration diagram of a light source apparatus according to a fifth embodiment of the present invention.

Figure 18 is a schematic configuration diagram of a light source apparatus according to a sixth embodiment of the present invention.

Figure 19 is a schematic configuration diagram of a light source according to an alternative embodiment of the sixth embodiment in which a light blocking member is provided.

Figure 20 is a schematic configuration diagram of a light source apparatus according to a seventh embodiment of the present invention.

Figure 21 is a schematic configuration diagram of a light source apparatus according to an eighth embodiment of the present invention.

DESCRIPTION OF THE PREFERRED EMBODIMENTS

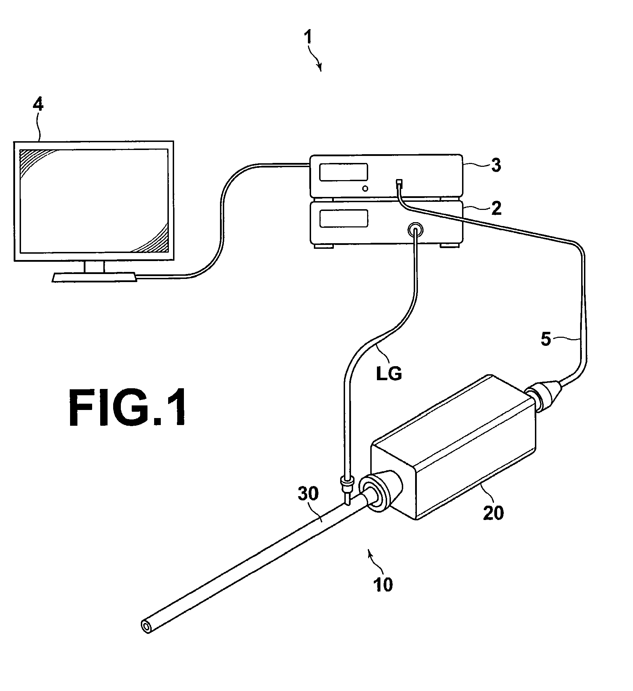

Hereinafter, a rigid endoscope system that employs a first embodiment of the light source apparatus of the present invention will be described in detail with reference to the accompanying drawings. The rigid endoscope system of the present embodiment has a characteristic feature in the configuration of the light source apparatus, but an overall system configuration will be described first. Figure 1 is an overview of the rigid endoscope system 1, illustrating a schematic configuration thereof.

As illustrated in Figure 1, the rigid endoscope system 1 of the present embodiment includes a light source apparatus 2 for emitting ordinary white light and excitation light, a rigid endoscope imaging device 10 for guiding and projecting ordinary light and excitation light emitted from the light source apparatus 2 to an examination area and capturing an ordinary image based on reflection light reflected from the examination area by the projection of the ordinary light and a fluorescence image based on fluorescence emitted from the examination area by the projection of the excitation light, a processor 3 for performing predetermined processing on an image signal obtained by the rigid endoscope imaging device 10, and a monitor 4 for displaying the ordinary image and fluorescence image of the examination area based on a display control signal generated by the processor 3.

As illustrated in Figure 1, the rigid endoscope imaging device 10 includes a body cavity insertion section 30 to be inserted into a body cavity and imaging unit 20 for capturing an ordinary image and a florescence image of an examination area guided by the body cavity insertion section 30.

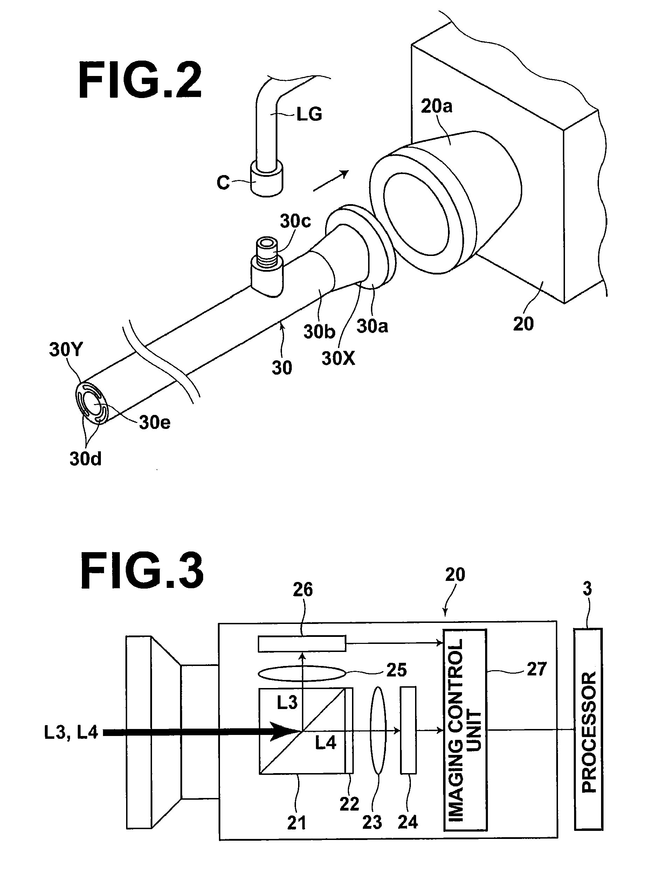

As shown in Figure 2, the body cavity insertion section 30 and imaging unit 20 are detachably connected. The body cavity insertion section 30 includes connection member 30a, insertion member 30b, a cable connection port 30c, projection windows 30d, and an imaging window 30e.

The connection member 30a is provided at a proximal end 30X of the body cavity insertion section 30 (insertion member 30b), and the imaging unit 20 and body cavity insertion section 30 are detachably connected by fitting connection member 30a into, for example, aperture 20a formed in the imaging unit 20.

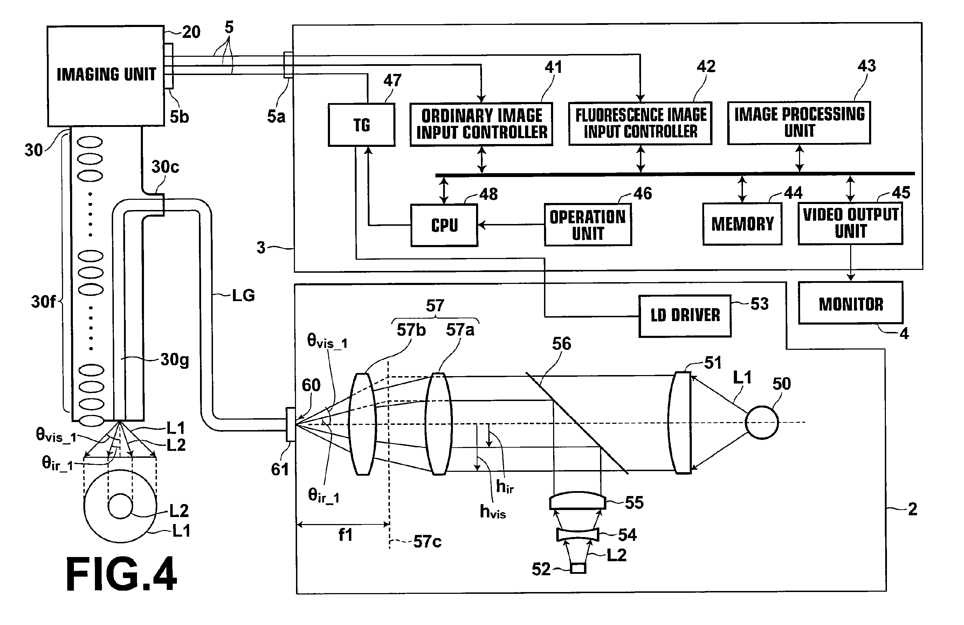

The insertion member 30b is a member to be inserted into a body cavity when imaging is performed in the body cavity. The insertion member 30b is formed of a rigid material and has, for example, a cylindrical shape with a diameter of about 5 mm. The body cavity insertion section 30 accommodates inside thereof relay lenses 30f (Figure 4) for forming an ordinary image and fluorescence image of an examination area entered from the imaging window 30e. The ordinary image and fluorescence image outputted from the relay lenses 30f are inputted to the imaging unit 20.

As illustrated in Figure 2, the cable connection port 30c is provided on the side surface of insertion member 30b and a light guide LG is mechanically connected to the port. This allows the light source apparatus 2 and insertion member 30b to be optically linked through the light guide LG.

The light guide LG is provided with a connector 61 (Figure 4) at a tip thereof and detachably connected to the light source apparatus 2 through the light guide LG. The light guide LG is constituted, for example, by a multimode optical fiber bundle.

The body cavity insertion section 30 includes inside thereof a multimode optical fiber bundle 30g for guiding ordinary light and excitation light outputted from the light guide LG connected to the cable connection port 30c. The multimode optical fiber bundle 30g guides the inputted ordinary light and excitation light to the distal end 30Y of the insertion member 30b and projects them onto an examination area. Tips of the multimode optical fibers 30g are polished to form the projection windows 30d.

Figure 3 illustrates a schematic configuration of the imaging unit 20. Imaging unit 20 includes a first imaging system for capturing a fluorescence image 4 of an examination area formed by the relay lenses 30f of the body cavity insertion section 30 and generating a fluorescence image signal of the examination area, and a second imaging system for capturing an ordinary image L3 of the examination area formed by the relay lenses of the body cavity insertion section 30 and generating an ordinary image signal of the examination area. These imaging systems are separated into two orthogonal optical axes by a dichroic prism 21 having a spectral characteristic that reflects the ordinary image L3 and transmits the fluorescence image L4.

The first imaging system includes excitation light cut filter 22 that cuts light having a wavelength not greater than that of the excitation light transmitted through the dichroic prism 21 and transmits fluorescence wavelength range illumination light to be described later, a first image forming optical system 23 that forms the fluorescence image L4 outputted from the body cavity insertion section 30 and transmitted through dichroic prism 21 and excitation light cut filter 22, and a high sensitivity image sensor 24 that captures the fluorescence image L4 formed by the first image forming optical system 23.

The high sensitivity image sensor 24 detects light in a wavelength range of the fluorescence image L4 with high sensitivity, converts the detected light to a fluorescence image signal, and outputs the fluorescence image signal. As for the high sensitivity image sensor 24, a monochrome image sensor may be used.

The second imaging system includes a second image forming optical system 25 that forms the ordinary image L3 outputted from the body cavity insertion section 30 and reflected from the dichroic prism 21, and an image sensor 26 that captures the ordinary image L3 formed by the second image forming optical system 25.

The image sensor 26 detects light in the wavelength range of ordinary image L3, converts the detected light to an ordinary image signal, and outputs the image signal. Color filters of three primary colors, red (R), green (G), and blue (B) or color filters of cyan (C), magenta (M), and yellow (Y) are arranged on the imaging surface of image sensor 26 in a Beyer or honeycomb pattern.

The imaging unit 20 includes an imaging control unit 27. The imaging control unit 27 performs CDS/AGC (correlated double sampling/automatic gain control) and A/D conversion on a fluorescence image signal outputted from the high sensitivity image sensor 24 and an ordinary image signal outputted from the image sensor 26, and outputs resultant image signals to the processor 3 through the cable 5 (Figure 1).

As illustrated in Figure 4, the processor 3 includes an ordinary image input controller 41, a fluorescence image input controller 42, image processing unit 43, memory 44, video output unit 45, operation unit 46, TG (timing generator) 47, and CPU 48.

Each of the ordinary image input controller 41 and fluorescence image input controller 42 is provided with a line buffer having a predetermined capacity. The ordinary image input controller 41 temporarily stores ordinary image signals with respect to each frame outputted from imaging control unit 27 of imaging unit 20 and the fluorescence image input controller 42 temporarily stores fluorescence image signals. Then, the ordinary image signals stored in the ordinary image input controller 41 and the fluorescence image signals stored in the fluorescence image input controller 42 are stored in memory 44 via the bus.

The image processing unit 43 receives an ordinary image signal and a fluorescence image signal with respect to each frame read out from the memory 44, performs predetermined processing on these image signals, and outputs the resultant image signals to the bus.

The video output unit 45 receives an ordinary image signal and a fluorescence image signal outputted from image processing unit 43 via the bus, generates a display control signal by performing predetermine processing on the received signals, and outputs the display control signal to monitor 4.

Operation unit 46 receives operator inputs, such as various types of operational instructions and control parameters. In the present embodiment, in particular, the operation unit 46 receives an instruction to change the exit angle of the excitation light or exit angle of the white light to be described in detail later.

The TG 37 outputs drive pulse signals for driving the high sensitivity image sensor 24 and image sensor 26 of imaging unit 20, and an LD driver 53 of the light source apparatus 2, to be described later. The CPU 48 performs overall control of the system.

As illustrated in Figures 1 and 4, the processor 3 is to be connected to the imaging unit 20 through the cable 5. The cable 5 includes signal wires for transferring an ordinary image signal and a fluorescence image signal captured by the imaging unit 20 and a control wire for sending a control signal outputted from the processor 3. The cable 5 is provided with connectors 5a and 5b at the tips thereof, and detachably connected to the processor 3 via the connector 5a and to the imaging unit 20 via the connector 5b.

As illustrated in Figure 4, the light source apparatus 2 includes a visible light lamp 50 (corresponding to second light source) for emitting ordinary light (white light) L1 which includes wavelengths of a broad range of 400 to 700 nm as diffused light, an aspherical lens for substantially collimating the ordinary light L1 emitted from the visible light lamp 50 and outputting the collimated light, a near infrared laser diode 52 (corresponding to firs light source) for emitting excitation light L2, which is near infrared light with a wavelength from 750 to 790 nm, an LD driver 53 for driving the near infrared laser diode 52, a magnification lens 54 for magnifying the excitation light L2 emitted from the near infrared laser diode 52 and outputs the magnified light, a collimating lens 55 for outputting the excitation light L2 outputted from the magnification lens as collimated light, a dichroic mirror 56 for transmitting the ordinary image L1 outputted from the aspherical lens 51 and reflecting the excitation light L2 outputted from the collimating lens 55 onto a zoom lens 57, to be described later, and the zoom lens 57 (corresponding to exit angle changing unit, incident angle changing optical system) for focusing the ordinary light L1 transmitted through the dichroic mirror 56 and the excitation light L2 reflected by the dichroic mirror 56 so as to incident on the light incident end face 60 of the light guide LG at a predetermined incident angle.

As for the visible light lamp 50, for example, a xenon lamp is used. In the present embodiment, white light is used as the ordinary light L1 (visible light), but the other light may also be used if it has a visible wavelength. Further, in the present embodiment, a near infrared laser diode is used as the light source for emitting the excitation light, but a near infrared light emitting diode may also be used.

As described above, the dichroic mirror 56 transmits the ordinary light L1 and reflects the excitation light L2, and disposed inclined by 45° with respect to the optical axis direction of the zoom lens 57.

The near infrared laser diode 52, magnification lens 54, and collimating lens 55 are disposed such that the direction of the excitation light L2 outputted therefrom is orthogonal to the optical axis of the zoom lens 57.

The zoom lens 57 includes a first movable lens 57a and a second movable lens 57b, and these lenses are moved in optical axis directions to change the focal length of the zoom lens 57. The change in the focal length of the zoom lens 57 causes a change in the incident angles of the inputted ordinary light L1 and excitation light L2 on the light incident end face 60 of the light guide LG, which, in turn, causes a change in the exit angles of the ordinary light L1 and excitation light L2 projected from the distal end of the body cavity insertion section 30 to the examination area. In the present embodiment, the zoom lens 57 includes two movable lenses, but the zoom lens 57 may includes three or more lenses. The "focal length of the zoom lens 57" as used herein refers the distance from the principal point 57c of the combined lenses constituting the zoom lens 57 to the light incident end face 60 of the light guide LG, as illustrated in Figure 4.

The relationship between the change in incident angle of the ordinary light L1 and the change in incident angle of the excitation light caused by the change in focal length of the zoom lens 57 will now be described.

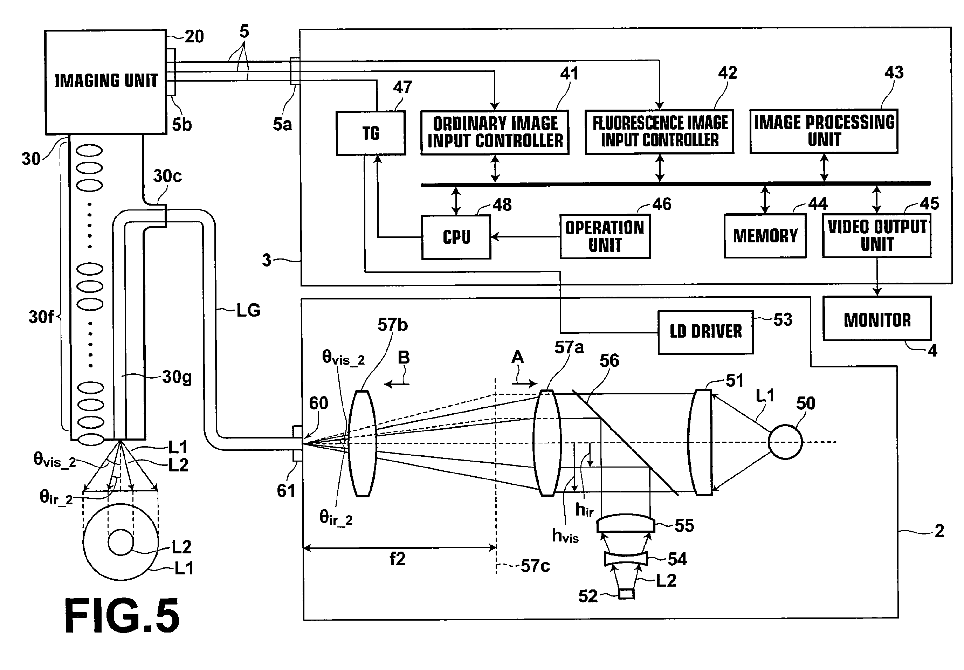

It is assumed here that the change in focal length of the zoom lens 57 from f1 shown in Figure 4 to f2 in Figure 5 causes, for example, a change in incident angle of the excitation light L2 from $\theta_{ir\_1}$ to $\theta_{ir\_2}$ and the change in incident angle of the ordinary light L1 from $\theta_{vis\_1}$ to $\theta_{vis\_2}$. As the incident height $h_{vis}$ of the ordinary light L1 and the incident height $h_{ir}$ of the excitation light L2 on the zoom lens 57 are constant, the following formula may be derived.

$$f_1 \tan\theta_{ir\_1} = f_2 \tan\theta_{ir\_2} = h_{ir}, \; f_1 \tan\theta_{vis\_1} = f_2 \tan\theta_{vis\_2} = h_{vis}$$

Then, from the formula given above, the relationship between the incident angle $\theta_{ir\_2}$ of the excitation light L2 and the incident angle $\theta_{vis\_2}$ of the ordinary light L1 after the change may be represented by the formula given below.

$$\frac{\tan\theta_{ir\_1}}{\tan\theta_{vis\_1}} = \frac{\tan\theta_{ir\_2}}{\tan\theta_{vis\_2}} = \frac{h_{ir}}{h_{vis}} = const.$$

$$\tan\theta_{ir\_2} = \frac{h_{vis}}{h_{ir}}\tan\theta_{vis\_2} = \frac{\tan\theta_{ir\_1}}{\tan\theta_{vis\_1}}\tan\theta_{vis\_2}$$

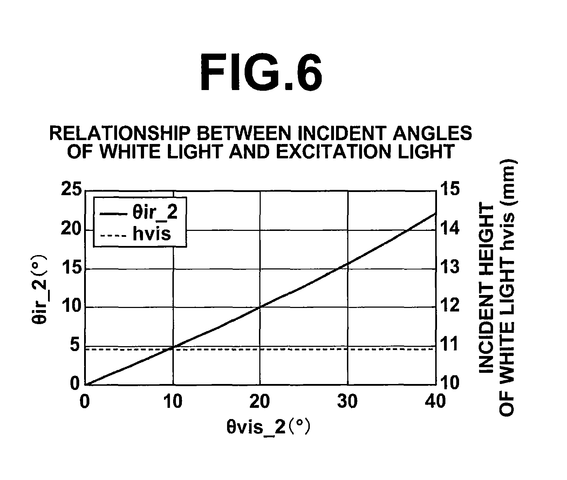

If, for example, $h_{ir}$=5.3 mm, $h_{vis}$=10.9 mm (incident angle $\theta_{ir\_1}$=10° and incident angle $\theta_{vis\_1}$=20°), the relationship between the incident angle $\theta_{ir\_2}$ of the excitation light L2 and incident angle $\theta_{vis\_2}$ of the ordinary light L1 may become like that represented by the solid line in Figure 6. Figure 6 also indicates the magnitude of the incident height $h_{vis}$ of the ordinary light L1 (white light) by the dashed line in order to make comparison with a second embodiment, to be described later. As described above, the incident height $h_{vis}$ is constant in the first embodiment.

As shown in Figure 6, the incident angle $\theta_{ir\_2}$ of the ordinary light L1 and the incident angle $\theta_{ir\_2}$ may be changed simultaneously with a predetermined relationship therebetween by changing the focal length of the zoom lens 57.

If the incident angle of light incident on an optical fiber is taken as $\theta_{in}$ and the exit angle of the light is taken as $\theta_{out}$, the relationship represented by formula given below generally holds true.

$$\theta_{out} = \theta_{in} \ (\theta_{in} \le \theta_{max})$$

$$\theta_{out} = \theta_{max} \ (\theta_{in} > \theta_{max})$$

where, $\theta_{max}$ = arcsin (NA) and NA is a numerical aperture of the optical fiber. In the present embodiment, the following are assumed: NA = 0.64 (possible value of glass optical fiber light guide that transmits wavelengths in the range from visible to near infrared wavelengths); $\theta_{in} \le \theta_{max}$ = 40°; and the incident angle on the optical fiber is equal to the exit angle, that is, the incident angle of the light incident on the light guide LG is equal to the exit angle of light exiting from light guide LG and multimode optical fibers 30g in the body cavity insertion section 30.

Then, under the aforementioned assumptions, if the distance between a planar object and the tip of the distal end of the body cavity insertion section 30 is taken as "d", radius of projection range of the ordinary light L1 and radius of projection range of the excitation light L2 before change in focal length of the zoom lens 57 are taken as $r_{vis\_1}$ and $R_{ir\_1}$ respectively, and radius of projection range of the ordinary light L1 and radius of projection range of the excitation light L2 after change in focal length of the zoom lens 57 are taken as $r_{vis\_2}$ and $R_{ir\_2}$ respectively, the relationship represented by the formula given below is obtained.

$$\frac{r_{ir\_2}}{r_{vis\_2}} = \frac{d\tan\theta_{ir\_2}}{d\tan\theta_{vis\_2}} = \frac{d\tan\theta_{ir\_1}}{d\tan\theta_{vis\_1}} = \frac{r_{ir\_1}}{r_{vis\_1}}$$

Thus, in the method of controlling the exit angles of the ordinary light L1 and excitation light L2 of the first embodiment, the ratio between the radius of projection range of the ordinary light L1 and the radius of projection range of the excitation light L2 on a planar object may be maintained constant. That is, when examining a planar object, control of the exit angles of the ordinary light L1 and excitation light L2 by the arrangement of the first embodiment can be said to be effective to the feeling of the user.

In the present embodiment, the dichroic mirror 56 is used to reflect the excitation light L2 onto the zoom lens 57, but a simple mirror that reflects an arbitrary wavelength may be used instead of the dichroic mirror 56. When using such a mirror, a mirror with a size sufficiently small relative to the size of the aspherical lens 51 should be used. As the amount of light outputted from the aspherical lens 51 is sufficiently large, shading of the ordinary light L1 by the mirror is not problematic.

Further, in the present embodiment, near infrared light is used as the excitation light, but the excitation light is not limited to this and light having a narrower wavelength range than the ordinary light is used. In addition, the excitation light is not limited to light having the aforementioned wavelength range and is determined, as appropriate, according to the type of fluorescent pigment used or the type of living tissue for causing autofluorescence.

An operation of the rigid endoscope system of the present embodiment will now be described.

First, the connector C of the light guide LG connected to the light source apparatus 2 is connected to the cable connection port 30c of the insertion member 30b of the body cavity insertion section 30, and the connector 5b of the cable 5 connected to the processor 3 is connected to the imaging unit 20.

Then, after the power of the light source apparatus 2 is switched on, the body cavity insertion section 30 is inserted into a body cavity of a subject by the user and the distal end of the body cavity insertion section 30 is placed adjacent to the examination area.

Ordinary light L1 is emitted from the visible light lamp 50 of the light source apparatus 2, which is inputted to the zoom lens 57 after being substantially collimated by the aspherical lens 51 and inputted to the light incident end face 60 of the light guide LG by the zoom lens 57 at a predetermined incident angle.

Note that the focal length of the zoom lens 57 at this time has been set to a value according to the exit angles of the ordinary light L1 and excitation light L2 set by the user through the operation unit 46.

In the mean time, excitation light L2 is emitted from the near infrared laser diode 52 of the light source apparatus 2, which is inputted to the dichroic mirror 56 after transmitting through the magnification lens 54 and collimating lens 55.

The excitation light L2 inputted to the dichroic mirror 56 is reflected and inputted to the zoom lens 57 and inputted to the light incident end face 60 of the light guide LG by the zoom lens 57 at a predetermined incident angle.

Then, as described above, the ordinary light L1 and excitation light inputted to the light incident end face 60 of the light guide LG are guided by the light guide LG, inputted to the light incident end faces of the multimode optical fibers 30g, guided by the multimode optical fibers 30g, and projected onto the examination area from the distal end of the body cavity insertion section 30.

Then, an ordinary image, which is based on reflection light reflected from the examination area by the projection of the ordinary light L1, and a fluorescence image, which is based on fluorescence emitted from the examination area by the projection of the excitation light L2, are captured. Note that ICG is administered to the examination area in advance and fluorescence emitted from the ICG is captured.

More specifically, when capturing the ordinary image, an ordinary image L3, which is based on reflection light reflected from the examination area by the projection of the ordinary light L1 is inputted from a distal end face 30Y, guided by the relay lenses 30f in the insertion member 30b, and outputted to the imaging unit 20.

The ordinary image L3 inputted to the imaging unit 20 is reflected in a right angle direction toward the image sensor 26 by the dichroic prism 21, then formed on the imaging surface of the image sensor 26 by the second image forming optical system 25, and sequentially captured by the image sensor 26 at a predetermined frame rate.

Each ordinary image signal sequentially outputted from the image sensor 26 is subjected to CDS/AGC (correlated double sampling/automatic gain control) and A/D conversion in the imaging control unit 27 and sequentially outputted to the processor 3 through the cable 5.

Then, each ordinary image signal inputted to the processor 3 is temporarily stored in the ordinary image input controller 41 and then stored in the memory 44. The ordinary image signals read out from the memory 44 with respect to each frame are subjected to tone correction and sharpness correction in the image processing unit 43, and sequentially outputted to the video output unit 45.

The video output unit 45 generates a display control signal by performing predetermined processing on the inputted ordinary image signals and sequentially outputs display control signals to the monitor 4 with respect to each frame. The monitor 4 displays an ordinary image based on the inputted display control signals.

In the mean time, when capturing the fluorescence image, a fluorescence image L4, which is based on fluorescence emitted from the examination area by the projection of the excitation light L2 is inputted from a distal end face 30Y, guided by the relay lenses 30f in the insertion member 30b, and outputted to the imaging unit 20.

The fluorescence image L4 inputted to the imaging unit 20 is transmitted through the dichroic prism 21 and excitation light cut filter 22, then formed on the imaging surface of the high sensitivity image sensor 24 by the first image forming optical system 23, and sequentially captured by the high sensitivity image sensor 24 at a predetermined frame rate.

Each fluorescence image signal sequentially outputted from the high sensitivity image sensor 24 is subjected to CDS/AGC (correlated double sampling/automatic gain control) and A/D conversion in the imaging control unit 27 and sequentially outputted to the processor 3 through the cable 5.

Then, each fluorescence image signal inputted to the processor 3 is temporarily stored in the fluorescence image input controller 42 and then stored in the memory 44. The ordinary image signals read out from the memory 44 with respect to each frame are subjected to predetermined processing in the image processing unit 43, and sequentially outputted to the video output unit 45.

The video output unit 45 generates a display control signal by performing predetermined processing on the inputted fluorescence image signals and sequentially outputs display control signals to the monitor 4 with respect to each frame. The monitor 4 displays a fluorescence image based on the inputted display control signals.

Then, after displaying the fluorescence image in the manner described above, if the user desires to change the exit angle of the ordinary light L1 or excitation light L2, an instruction to change the exit angle of the ordinary light L1 or excitation light L2 is set and entered in the operation unit 46.

Then, the first and second movable lenses 57a, 57b are moved according to the exit angle of the ordinary light L1 or excitation light L2 set and entered in the operation unit 46, whereby the focal length of the zoom lens 57 is changed. This causes incident angles of the ordinary light L1 and excitation light L2 on the light incident end face 60 of the light guide LG to be changed, whereby the exit angles of the ordinary light L1 and excitation light L2 with respect to the examination area are changed at the same time.

According to the rigid endoscope system of the present embodiment, the exit angles of the ordinary light L1 and excitation light L2 with respect to the examination area are changed simultaneously by the zoom lens 57. This allows the radio between projection ranges of the ordinary light L1 and excitation light L2 to be set to a desired value without requiring a complicated procedure, as well as maintaining sufficient biological surface illuminance of both the ordinary light L1 and excitation light L2.

Rigid endoscope systems that employ light source apparatuses according to second to eighth embodiments of the present invention will now be described. The rigid endoscope systems that employ light source apparatuses according to second to eighth embodiments differ from the rigid endoscope system of the first embodiment described above only in the configuration of the light source apparatuses, so that, hereinafter, only the configuration of each light source apparatus will be described.

In the light source apparatus 2 of the first embodiment, the ratio between the radii of projection ranges of the ordinary light L1 and excitation light L2 on the examination area is maintained constant as described above. In the case where the examination area is not a planar obj ect but an obj ect having a shape close to an incurved surface, such as the inner layer of peritoneum, however, it is more effective to the feeling of the user to maintain the ratio between exit angles of the ordinary light L1 and excitation angle L2 constant rather than maintaining the ratio between the radii of projection ranges of the ordinary light L1 and excitation light L2 constant. A light source apparatus 6 according to the second embodiment performs such control of the exit angles of the ordinary light L1 and excitation light L2.

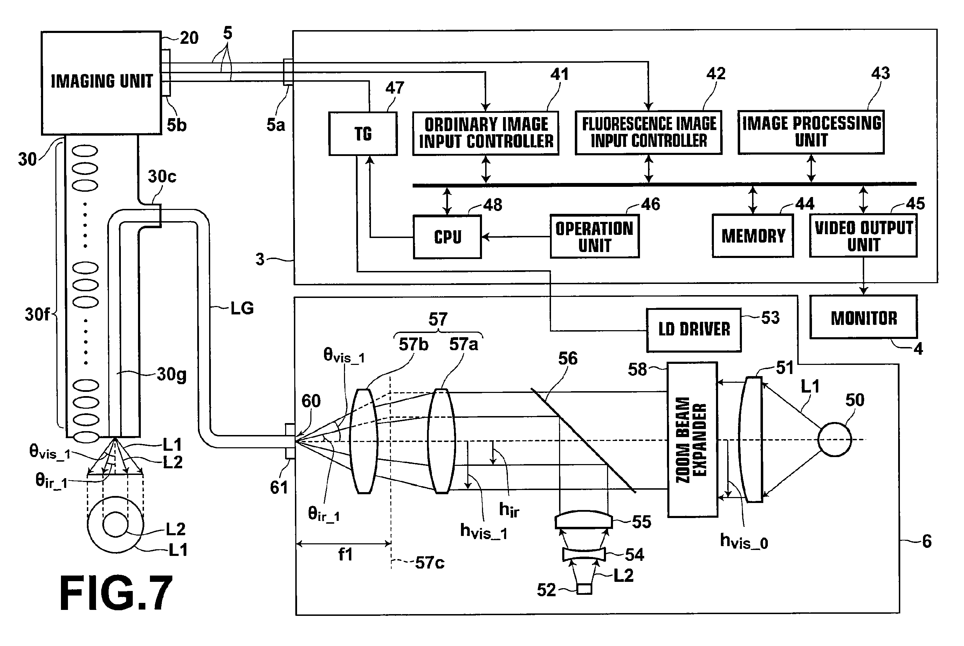

More specifically, as illustrated in Figure 7, the light source apparatus 6 of the second embodiment includes a zoom beam expander 58 (corresponding to the upstream optical system) between the aspherical lens 51 and dichroic mirror 56. The zoom beam expander 58 is a lens system that converts inputted collimated light to another collimated light having a different diameter. In the light source apparatus 6 of the second embodiment, the incident height of the ordinary light L on the zoom lens 57 is changed by the zoom beam expander 58 along with the change in the focal length of the zoom lens 57 in order to perform control such that the ratio between exit angles of the ordinary light L1 and excitation light L2 is maintained constant. By changing the incident height of the ordinary light L1 on the zoom lens 57 in the manner described above, only the incident angle of the ordinary light L1 on the light incident end face 60 of the light guide may be changed. Note that the zoom beam expander itself is a known optical component and, for example, such a zoom beam expander available from Melles Griot as described in http://www.mgkk.com/products/pdf/02-08-laseracce/0208-6.pdf may be used.

Hereinafter, a description will be made of a case in which the exit angle of the excitation light L2 and exit angle of the ordinary light L1 are changed at the same time by changing the focal length of the zoom lens 57 and the incident height $h_{vis}$ of the ordinary light L1 on the zoom lens 57 under the restrictions that the incident height $h_{ir}$ of the excitation light L2 on the zoom lens 57 is constant and the ratio "k" of the exit angle of the excitation light L2 to the exit angle of the ordinary light L1 is constant.

If it is assumed that the change in focal length of the zoom lens 57 from f1 to f2 and change in incident height of the ordinary light L1 on the zoom lens 57 from $h_{vis1}$ to $h_{vis2}$ causes a change in incident angle of excitation light L2 from $\theta_{ir\_1}$ to $\theta_{ir\_2}$ and a change in incident angle of the ordinary light L1 from $\theta_{vis\_1}$ to $\theta_{vis\_2}$ on the light incident end face 60 of the light guide LG, the relationship represented by the formula given below may be derived.

$$f_1 \tan\theta_{vis\_1} = h_{vis\_1}, \quad f_2 \tan\theta_{vis\_2} = h_{vis\_2}$$

$$f_1 \tan\theta_{ir\_1} = f_2 \tan\theta_{ir\_2} = const. = h_{ir}$$

$$\frac{\theta_{ir\_2}}{\theta_{vis\_2}} = \frac{\theta_{ir\_1}}{\theta_{vis\_1}} = const. = k$$

From the relationship of the formula given above, the value $h_{ivs\_2}$ of incident height of the ordinary light L1 that should be satisfied may be represented by the formula given below.

$$h_{vis\_2} = h_{vis\_1} \frac{\tan(k\theta_{vis\_1})}{\tan\theta_{vis\_1}} \frac{\tan\theta_{vis\_2}}{\tan(k\theta_{vis\_2})} = f_1 \tan(k\theta_{vis\_1}) \frac{\tan\theta_{vis\_2}}{\tan(k\theta_{vis\_2})} = h_{ir} \frac{\tan\theta_{vis\_2}}{\tan(k\theta_{vis\_2})}$$

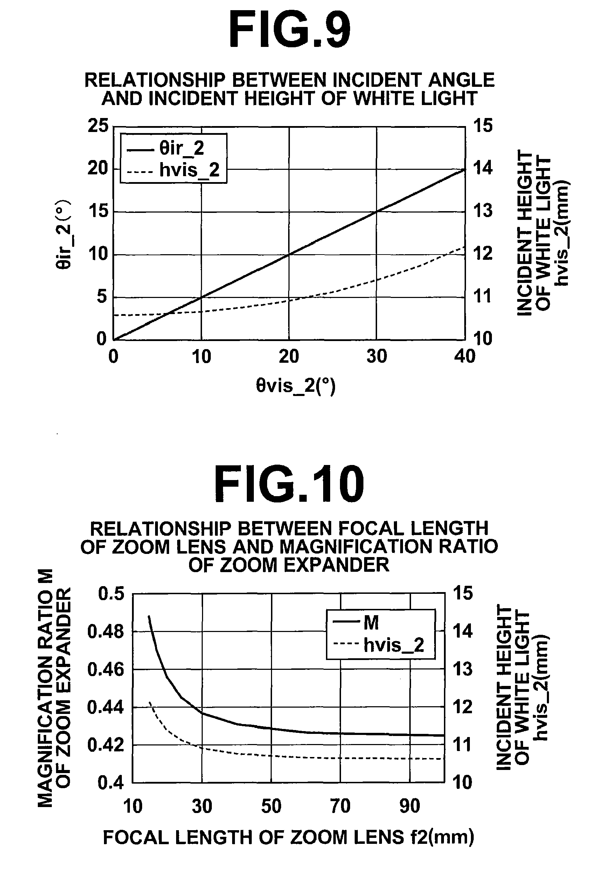

In the case, for example, where the ratio "k" of the exit angle of the excitation light L2 to the exit angle of the ordinary light L1 is desired to be maintained at k=1/2, if $h_{ir}$ = 5.3 mm (incident angle of the excitation light L2 prior to change is $\theta_{ir\_1}$ = 10°, incident angel of the ordinary light L1 is $\theta_{vis\_1}$ = 20°, and incident height of the ordinary light prior to change is $h_{vis\_1}$ = 10.9 mm), incident height $h_{vis\_2}$ of the ordinary light L1 on the zoom lens 57 with respect to the exit angle $\theta_{vis\_2}$ of the ordinary light L1 is like that shown by the dashed line in Figure 9. Figure 9 also indicates the relationship between $\theta_{vis\_2}$ and $\theta_{ir\_2}$ by the solid line. As the ratio is constant, the relationship becomes linear as shown in Figure 9.

Further, the relationship between the focal length f2 of the zoom lens 57 and incident height $h_{vis\_2}$ of the ordinary light L1 after change is like that represented by the formula given below.

$$f_2 = \frac{h_{ir}}{\tan\theta_{ir\_2}} = \frac{h_{ir}}{\tan(k\theta_{vis\_2})} = \frac{h_{ir}}{\tan\{k\arctan(h_{vis\_2}/f_2\}}$$

Thus, the following may be derived.

$$h_{vis\_2} = f_2 \tan\{(1/k)\arctan(h_{ir}/f_2)\}$$

In the mean time, the properties of the zoom beam expander 58 may be represented by the formula given below. Where M is multiplication ratio of the zoom beam expander 58 and $h_{vis\_0}$ is the incident height of the ordinary light L1 on the zoom beam expander 58.

$$h_{vis\_2} = M h_{vis\_0}$$

Based on the two formulae given above, $h_{vis\_2}$ may be eliminated and the following may be derived.

$$M = (f_2 / h_{vis\_0})\tan\{(1/k)\arctan(h_{ir}/f_2)\}$$

Thus, by setting the magnification ratio M of the zoom beam expander 58 along with the setting of the focal length f2 of the zoom lens 57 according to the formula given above, simultaneous control may be achieved in which the ratio between the exit angle of the ordinary light L1 and exit angle of the excitation light L2 is maintained constant.

By way of example, Figure 10 illustrates, by solid line, the relationship between the focal length f2 of the zoom lens 57 and magnification ratio M of the zoom beam expander 58. Figure 10 also indicates, by dashed line, a variation in the incident height $h_{vis\_2}$ of the ordinary light L1.

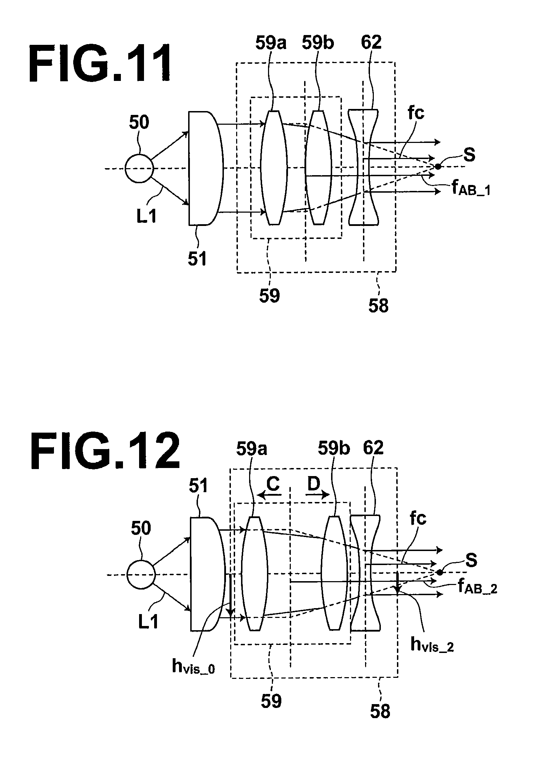

As described above, in the light source apparatus 6 of the second embodiment, any known zoom beam expander may be used as the zoom beam expander 58 but, for example, a zoom beam expander configured in the manner illustrated in Figure 11 may also be used.

More specifically, the zoom beam expander 58 illustrated in Figure 11 includes a zoom lens 59 having two movable lenses 59a, 59b and a concave lens 62 that receives the ordinary light L1 outputted from the zoom lens 59.

The zoom beam expander 58 shown in Figure 11 may change the incident height $h_{vis}$ of the ordinary light L1 by changing the focal length of the zoom lens 59 such that the focal point of the zoom lens 59 always coincides with the focal point S. Figure 12 illustrates the case where the focal length of the zoom lens 59 is changed from $f_{AB\_1}$ to $f_{AB\_2}$ by moving the movable lenses 59a and 59b in the directions of arrows C and D respectively and the incident height of the ordinary light L1 is changed smaller than that of Figure 11.

In the case where the zoom beam expander 58 is configured in the manner illustrated in Figures 11 and 12, the properties thereof may be represented by the formula given below, in which $f_c$ represents the focal length of the concave lens 62.

$$h_{vis\_2} = Mh_{vis\_0} = \frac{f_C}{f_{AB\_2}} h_{vis\_0}$$

The relationship between the focal length f2 in the latter stage and incident height $h_{vis\_2}$ of the ordinary light L1 is represented by the formula given below as described above.

$$h_{vis\_2} = f_2 \tan\{(1/k)\arctan(h_{ir}/f_2)\}$$

Using the two formulae, the $h_{vis\_2}$ may be eliminated and the following may be derived.

$$f_{AB\_2} = h_{vis\_0} \frac{f_C}{f_2 \tan\{(1/k)\arctan(h_{ir}/f_2)\}}$$

Thus, by setting the focal length $f_{AB\_2}$ of the zoom lens 59 of the zoom beam expander 58 along with setting the focal length f2 of the zoom lens 57 according to the formula given above, simultaneous control may be achieved in which the ratio between the exit angle of the ordinary light L1 and exit angle of the excitation light L2 is maintained constant.

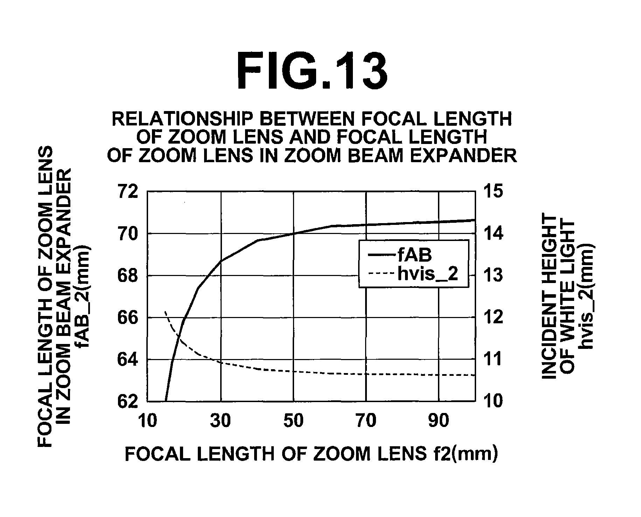

By way of example, Figure 13 illustrates, by the solid line, the relationship between the focal length f2 of the zoom lens 57 and the focal length $f_{AB}$ of the zoom lens 59 in the zoom beam expander 58 when $h_{vis\_0}$ = 25 mm and $f_c$ = 30 mm. Figure 13 also indicates, by the dashed line, a variation in the incident height $h_{vis\_2}$ of the ordinary light L1.

As described above, in the light source apparatus 6 of the second embodiment, the incident height of the ordinary light L1 on the zoom lens 57 is changed using the zoom beam expander 58, but the configuration for changing the incident height of the ordinary light L1 is not limited to this, and other optical systems, such as an aperture and the like may be used for this purpose.

In the light source apparatuses 2 and 6 according to the first and second embodiments, an arrangement is adopted in which the ordinary light L1 emitted from the visible light lamp 50 is guided parallel to the optical axis of the zoom lens 57 while the excitation light L2 emitted from the near infrared laser diode 52 is guided perpendicular to the optical axis of the zoom lens 57. Alternatively, an arrangement may be adopted in which a dichroic mirror that transmits the excitation light L2 and reflects the ordinary light L1 is used as the dichroic mirror 56 and the excitation light is guided parallel to the optical axis of the zoom lens 57 while the ordinary light L1 is guided perpendicular to the optical axis of the zoom lens 57. In the case where this arrangement is employed in the light source apparatus 6 of the second embodiment, the zoom beam expander 58 is disposed behind the near infrared laser diode 52, and the incident height of the excitation light L2 is changed along with the change in focal length of the zoom lens 57.

In the light source apparatuses 2 and 6 according to the first and second embodiments, the zoom lens 57 is used for changing the incident angles of the ordinary light L1 and excitation light L2. But, a varifocal lens capable of changing the focal length as in the zoom lens 57 may also be employed.

As described above, when changing the focal length of the zoom lens 57, the focal position thereof is maintained at the light incident end face 60 of the light guide LG by moving both the first and second movable lenses 57a and 57b.

In contrast, although the varifocal lens includes two convex lenses as in the zoom lens 57, when changing the focal length, the lens on the side of the light guide LG is fixed and only the other lens is moved.

In the case where the varifocal lens is used, therefore, the change in focal length accompanies a focal position shift to the upstream side or downstream side of the light incident end face, but the exit angles of the ordinary light L1 and excitation light L2 outputted from the tip of the body cavity insertion section 30 are identical to those when the zoom lens 57 is used. As described above, in the case where the varifocal lens is used, the focal position is displaced from the position on the light incident end face 60, but the displacement of the focal position allows the number of multimode optical fibers of the light guide LG to which the excitation light L2 is inputted to be increased in comparison with the case where the zoom lens 57 is used. That is, bright points on the output face at the tip of the body cavity insertion section 30 may be dispersed which is advantageous from the viewpoint of laser safety.

Still further, in the light source apparatus 6 according to the second embodiment, the zoom beam expander 58 is controlled along with the change in focal length of the zoom lens 57 such that the ratio between the exit angle of the ordinary light L1 and the exit angle of the excitation light L2 is maintained constant. But the invention is not limited to this and, for example, the zoom beam expander 58 may be controlled along with the change in focal length of the zoom lens 57 such that only the exit angle of the excitation light L2 is changed while the exit angle of the ordinary light L1 is maintained constant. Such control allows only the exit angle of excitation light L2 to be changed.

Preferably, an excitation light independent change mode in which only the exit angle of the excitation light L2 is changed as described above and a simultaneous change mode in which the exit angles of the ordinary light L1 and excitation light L2 are changed simultaneously as described in the second embodiment be made switchable. For example, a selection instruction from the user may be accepted via the operation unit 46 and switching may be performed between the simultaneous change mode and excitation light independent change mode according to the selection instruction.

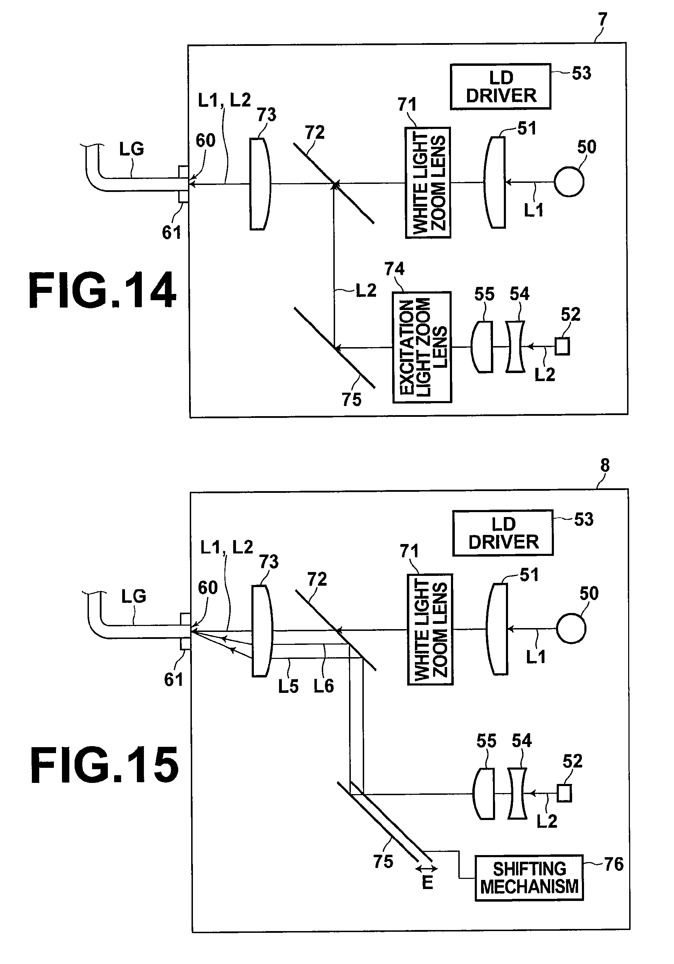

A light source apparatus according to a third embodiment of the present invention will now be described. In the first and second embodiments described above, incident angles of the ordinary light L1 and excitation light L2 on the light incident end face 60 of the light guide LG is changed simultaneously using one zoom lens 57. But the invention is not limited to this, and the incident angle of the ordinary light L1 and the incident angle of the excitation light L2 may be changed by providing two zoom lenses, one of which is a white light zoom lens 71 (corresponding to the second light exit angle change section) for changing the incident angle of the ordinary light L1 and the other of which is an excitation light zoom lens 74 (corresponding to the first light exit angle change section) for changing the incident angle of the excitation light L2, as in a light source apparatus 7 illustrated in Figure 14. More specifically, in the light source apparatus 7 of the third embodiment, the ordinary light L1 emitted from the visible light lamp 50 is inputted to the white light zoom lens 71 after being changed to substantially parallel light by the aspherical lens 51 and inputted to a condenser lens 73 after being transmitted through the white light zoom lens 71 set to a predetermined focal length (incident angle) and a dichroic mirror 72.

In the mean time, the excitation light L2 emitted from the near infrared laser diode 52 is inputted to the excitation light zoom lens 74 after being transmitted through the magnification lens 54 and collimating lens 55, reflected by a mirror 75 toward the dichroic mirror after being transmitted through the excitation light zoom lens 74 set to a predetermined focal length (incident angle), reflected by the dichroic mirror 72 toward the condenser lens 73, and inputted to the condenser lens 73.

The ordinary light L1 inputted to the condenser lens 73 is inputted to the light incident end face 60 of the light guide LG at an incident angle adjusted by the white light zoom lens 71 and the excitation light L2 is inputted to the light incident end face 60 of the light guide LG at an incident angle adjusted by the excitation light zoom lens 74.

Note that the incident angles of the ordinary light L1 and excitation light L2 may be adjusted such that the ratio between the radius of the projection range of the ordinary light L1 and the radius of the projection range of the excitation light L2 at an examination area is constant, as in the first embodiment described above, or such that the ratio between the exit angles of the ordinary light L1 and excitation light L2 at the examination area is constant by changing the incident angle of the ordinary light L1 along with the change in incident angle of the excitation light L2, as in the second embodiment.

Next, a light source apparatus according to a fourth embodiment will be described. In the light source apparatus 7 according to third embodiment described above, the incident angle of the excitation light L2 is adjusted by the excitation light zoom lens 74. But, as in a light source 8 illustrated in Figure 15, the incident angle of the excitation light L2 on the light incident end face 60 of the light guide LG may be changed by moving the mirror 75 in the arrow directions E to change the reflection position of the excitation light L2 on the dichroic mirror 72, thereby changing the incident position of the excitation light L2 on the condenser lens 73. The term "incident angle of the excitation light L2" as used herein refers to an angle of the optical axis of the excitation light L2 with respect to the optical axis of the light guide LG.

More specifically, if the mirror 75 is positioned at a place closer to the near infrared laser diode 52 shown in Figure 15, the excitation light L2 is inputted to the condenser lens 73 as excitation light L5 while if the mirror 75 is positioned at a place closer to the condenser lens 73 shown in Figure 15, the excitation light L2 is inputted to the condenser lens 73 as excitation light L6. That is, the closer the mirror 75 to the near infrared laser diode 52, the more remote is the incident position of the excitation light L2 on the condenser lens 73 from the optical axis thereof.

If the incident position of the excitation light on the condenser lens 73 is changed as described above, the incident angle of the excitation light on the light incident end face 60 of the light guide LG is changed. More specifically, as illustrated in Figure 15, the excitation light L5 has a larger incident angle than that of the excitation light L6 with respect to the direction perpendicular to the light incident end face 60.

By changing the incident angle of the excitation light on the light incident end face 60 of the light guide LG in the manner described above, the exit angle may be changed according to the value of the incident angle. That is, as illustrated in Figure 15, the excitation light L5 having a larger incident angle with respect to the direction perpendicular to the light incident end face 60 of the light guide LG will have a broader exit angle than that of the excitation light L6.

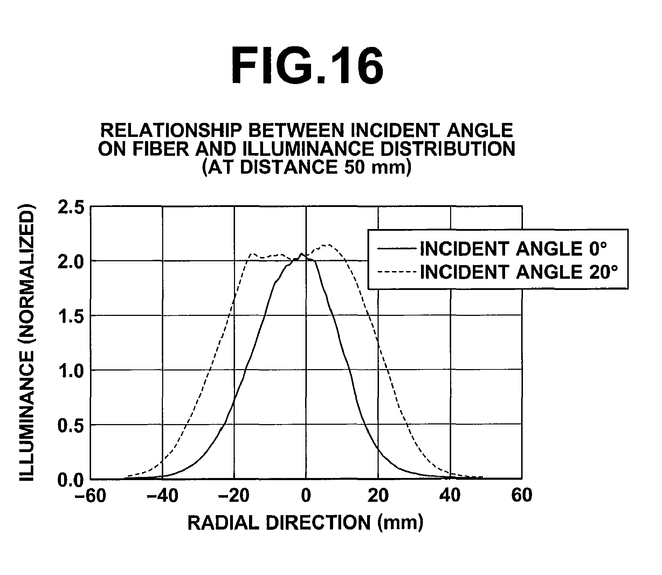

By changing the incident angle of the excitation light on the light incident end face 60 of the light guide LG in the manner described above, the projection range of the excitation light at the same distance may be changed, that is, a graph illustrating experimental data that indicate that the exit angle is changed is shown in Figure 16. Figure 16 shows experimental data of an illuminance distribution when the excitation light is incident on the light incident end face 60 of the light guide LG from a direction inclined by an incident angle of 20° with respect to a direction perpendicular to the light incident end face 60 of the light guide LG and of an illuminance distribution when the excitation light is incident on the light incident end face 60 of the light guide LG from a direction perpendicular to the light incident end face 60 of the light guide LG. As shown in Figure 16, in the case where the excitation light is incident on the light incident end face 60 of the light guide from a direction inclined by an incident angle of 20° with respect to a direction perpendicular to the light incident end face 60 of the light guide LG, the excitation light propagating through the multimode optical fibers of the light guide LG and exiting therefrom forms two illuminance distributions, each having a peak. The two illuminance distributions overlap only partially and a relatively broad illuminance distribution may be formed. Too large incident angle, however, may result in that the distance between the two illuminance distributions becomes large with reduced illuminance, whereby excitation light having sufficient illuminance can not be projected. Therefore, it is preferable that the incident angle be set to a vale less than a predetermined upper limit.

Although Figure 15 indicates two angles as the incident angles on the light incident end face 60 of the light guide LG, that is, two positions as the positions of the mirror 75. The invention is not limited to this and the amount and direction (to the condenser lens 73 or visible light lamp 50) of the shift may be set arbitrarily by the user through the operation unit 46 of the processor 3.

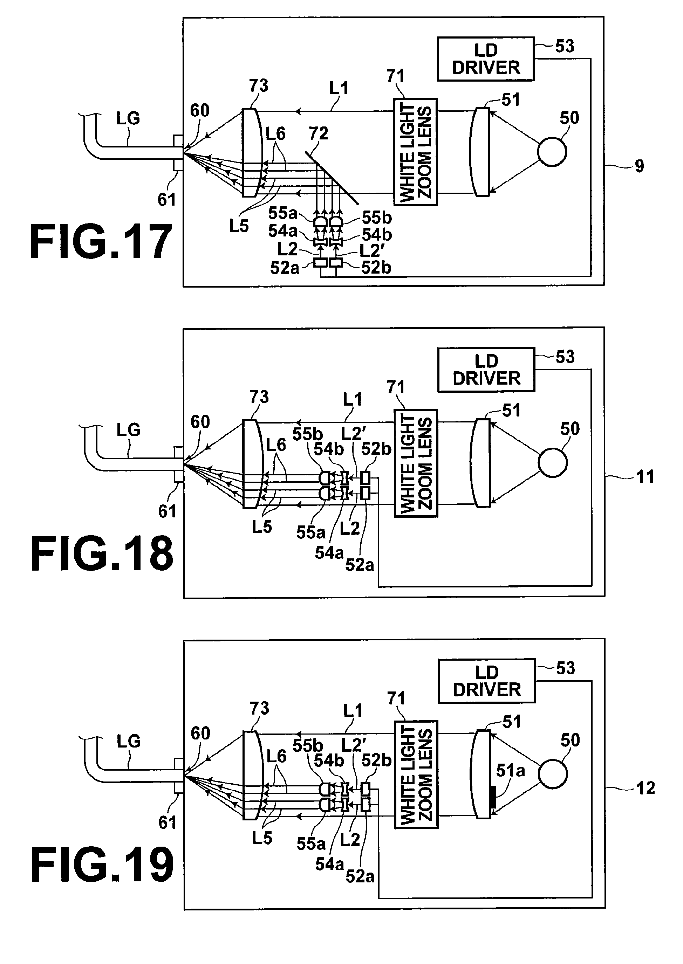

A light source apparatus according to a fifth embodiment of the present invention will now be described. In the light source apparatus 8 according to the fourth embodiment described above, the incident position of the excitation light on the condenser lens 73 is changed by shifting the mirror 75. In the light source apparatus 9 of the fifth embodiment, a set of near infrared laser diode 52a, magnification lens 54a, and collimating lens 55a and a set of near infrared laser diode 52b, magnification lens 54b, and collimating lens 55b are disposed in the directions of the optical axis of the condenser lens 73, and emission of excitation light L2 from the near infrared laser diode 52a and emission of excitation light L2' from the near infrared laser diode 52b are switched by an LD driver 53, as illustrated in Figure 17.

In the case where emission of excitation light L2 from the near infrared laser diode 52a and emission of excitation light L2' from the near infrared laser diode 52b are switched as described above, the reflection position of the excitation light on the dichroic mirror 72 may also be changed, as in the light source apparatus 8 of the fourth embodiment. This may change the incident position of the excitation light on the condenser lens 73, which may, in turn, change the incident angle of the excitation light on the light incident end face 60 of the light guide LG, whereby the exit angle of the excitation light may be changed. In the fifth embodiment illustrated in Figure 1, two sets of near infrared laser diodes, magnification lenses and collimating lenses are provided. But, in the case where the exit angle of the excitation light is to be changed three steps or more, the number of sets of near infrared laser diodes, magnification lenses and collimating lenses may be increased according to the exit angle steps and these sets of components may be disposed in the directions of the optical axis of the condenser lens 73.

Instead of providing a plurality of sets of near infrared laser diodes, magnification lenses and collimating lenses, as in the light source apparatus 9 of the fifth embodiment, one set of near infrared laser diode, magnification lens, and collimating lens may be shifted by a predetermined shifting mechanism in the directions of the optical axis of the condenser lens 73 to change the reflection position of the excitation light on the dichroic mirror 72.

Next, a light source apparatus according to a sixth embodiment of the present invention will be described. In the light source apparatus according to the fourth or fifth embodiment, the excitation light emitted from the near infrared laser diode is inputted to the condenser lens 73 by reflecting the excitation light at right angle by the dichroic mirror 72. In a light source apparatus 11 according to the sixth embodiment, a set of near infrared laser diode 52a, magnification lens 54a, and collimating lens 55a and a set of near infrared laser diode 52b, magnification lens 54b, and collimating lens 55b are disposed on the optical path of the ordinary light L1 in the directions orthogonal to the optical axis of the condenser lens 73, without the dichroic mirror 72, and emission of excitation light L2 from the near infrared laser diode 52a and emission of excitation light L2' from the near infrared laser diode 52b are switched by the LD driver 53, as illustrated in Figure 18.

Also in the light source apparatus 11 according to the sixth embodiment, instead of providing a plurality of sets of near infrared laser diodes, magnification lenses and collimating lenses, one set of near infrared laser diode, magnification lens and collimating lens may be shifted by a predetermined shifting mechanism in the directions orthogonal to the optical axis of the condenser lens 73 to change the reflection position of the excitation light on the condenser lens 73.

Note that the provision of the near infrared laser diodes, magnification lenses, and collimating lenses on the optical path of the ordinary light L1, as in the light source apparatus 11 of the sixth embodiment, does not cause insufficiency in the amount of the ordinary light L1 and any problematic shadow to be captured in the image of the examination area due to diffraction effect and diffusion effect.

Further, the shading of the ordinary light L1 by the near infrared laser diode does not cause any problem. For example, in the case where a precision aspherical lens with a diameter of 50 mm (available from Edmund) is used as the aspherical lens 51 and a laser diode with a diameter of 5.6 mm (available from Edmund) is used as the near infrared diode, the transmission efficiency is calculated as 1 - (5.6/50) = 99%.

Note that, however, the near infrared laser diode may become faulty due to heating by the ordinary light L1 as the near infrared laser diode is provided on the optical path of the ordinary light L1 in the sixth embodiment.

Consequently, a light blocking member 51a for blocking the ordinary light L1 toward the near infrared laser diode 52 may be provided on a portion of the surface of the aspherical lens 51 on the side of the visible light lamp 50, as illustrated in Figure 19. As for the light blocking member 51a, a member that reflects or absorbs the ordinary light may be used. In the case where a member that reflects the ordinary light L1 is used as the light blocking member 51a, it is preferable that the member be arranged so as to reflect the ordinary light in a direction other than the direction toward the visible light lamp 50.

Further, in the description above, the light blocking member 51a is provided on the surface of the aspherical lens 51 on the side of the near infrared laser diode 52, but the member may be provided on the surface of the aspherical lens 51 on the side of the near infrared laser diode 52 or at a position separated from the aspherical lens 51.

Alternatively, a reflection member that reflects the ordinary light L1 to the visible light lamp 50 side of the near infrared laser diode may be provided. It is also preferable that the reflection member be arranged so as to reflect the ordinary light L1 in a direction other than the direction toward the visible light lamp 50. Otherwise, a thermal insulation member may be provided for the near infrared laser diode instead of the reflection member. As for the thermal insulation member, any known material may be used. Still further, both the thermal insulation member and reflection member may be provided for the near infrared laser diode.

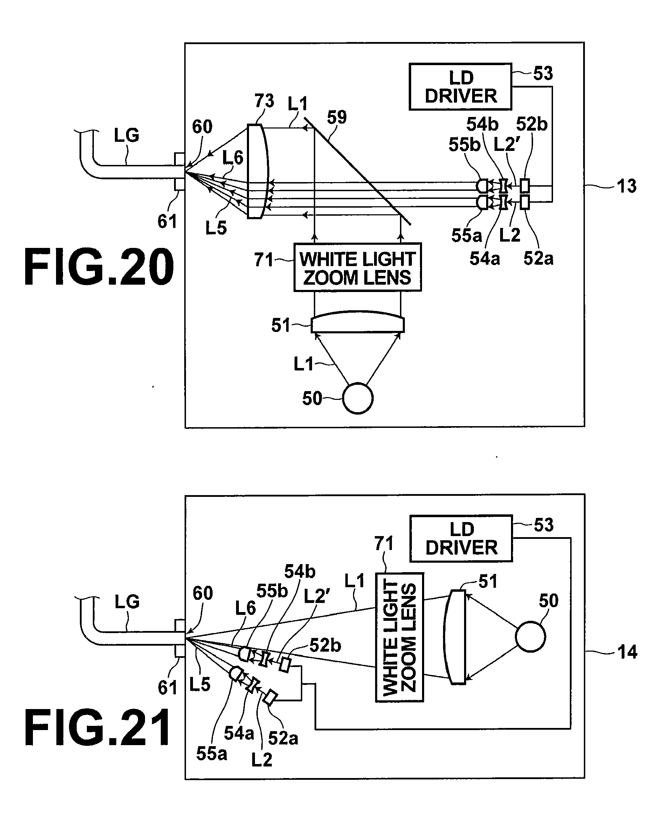

A light source apparatus according to a seventh embodiment of the present invention will now be described. In the light source apparatus according to the fifth embodiment, the visible light lamp 50 and aspherical lens 51 are disposed in an optical direction of the condenser lens 73 while the near infrared laser diodes 52a, 52b, magnification lenses 54a, 54b, and collimating lenses 55a, 55b are disposed in a direction orthogonal to the optical direction of the condenser lens 73. In contrast, in a light source apparatus 13 according to the seventh embodiment, the near infrared laser diodes 52a, 52b, magnification lenses 54a, 54b, and collimating lenses 55a, 55b are disposed in an optical direction of the condenser lens 73 while the visible light lamp 50 and aspherical lens 51 are disposed in a direction orthogonal to the optical direction of the condenser lens 73, as illustrated in Figure 20. The dichroic mirror 59 in the light source apparatus 13 shown in Figure 20 reflects the ordinary light L1 emitted from the visible light lamp 50 and transmitted through the aspherical lens 51 and white light zoom lens 71 toward the condenser lens 73 while transmits the excitation light L2 transmitted from the near infrared laser diode 52a or 52b.

Also in the light source apparatus 13 according to the seventh embodiment, the incident position of the excitation light on the condenser lens 73 may be changed by switching emission of the excitation light L2 from the near infrared diode 52a and emission of the excitation light L2' from the near infrared diode 52b by the LD driver 53.

Also in the light source apparatus 13 according to the seventh embodiment, one set of near infrared laser diode, magnification lens and collimating lens may be shifted by a predetermined shifting mechanism in the directions orthogonal to the optical axis of the condenser lens 73 to change the reflection position of the excitation light on the condenser lens 73, instead of providing a plurality of sets of near infrared laser diodes, magnification lenses, and collimating lenses.

Next, a light source apparatus according to an eighth embodiment of the present invention will be described. In the light source apparatus according to any of fifth to seventh embodiment, the excitation light emitted from the near infrared laser diode is condensed and inputted to the light incident end face 60 of the light guide LG by the condenser lens 73. In contrast, in a light source apparatus 14 according to the eighth embodiment, the excitation light L5 emitted from the near infrared laser diode 52a and transmitted through the magnification lens 54a and collimating lens 55a or the excitation light L6 emitted from the near infrared laser diode 52b and transmitted through the magnification lens 54b and collimating lens 55b is directly inputted to the light incident end face 60 of the light guide LG without providing the condenser lens 73, as illustrated in Figure 21.

Also in the light source apparatus 14 according to the eighth embodiment, the incident angle of the excitation light on the light incident end face 60 of the light guide LG may be changed by switching emission of the excitation light L2 from the near infrared diode 52a and emission of the excitation light L2' from the near infrared diode 52b by the LD driver 53.

Also in the light source apparatus 14 according to the eighth embodiment, one set of near infrared laser diode, magnification lens, and collimating lens may be shifted by a predetermined shifting mechanism to change the incident angle of the excitation light on the light incident end face 60 of the light guide LG, instead of providing a plurality of sets of near infrared laser diodes, magnification lenses and collimating lenses. Here, the set of near infrared laser diode, magnification lens, and collimating lens is shifted to a predetermined direction (circumferential direction of a circle with the center of the light incident end face 60 of the light guide LG as the center thereof) by a shifting mechanism such that the optical axis thereof is inclined at a plurality of angles with respect to the direction perpendicular to the light incident end face 60 of the light guide LG.

In the light source apparatuses according to the third to eighth embodiments, it is possible to independently control the exit angle of the ordinary light L1 and exit angle of the excitation light L2. But it is desirable that the simultaneous change mode of the exit angles of the ordinary light L1 and excitation light L2 and excitation light independent change mode in which the exit angle of the ordinary light L1 is maintained constant and only the exit angle of the excitation light L2 is changed as in the first and second embodiments be made switchable. For example, a selection instruction from the user may be accepted via the operation unit 46 and switching may be performed between the simultaneous change mode and excitation light independent change mode according to the selection instruction.

In the embodiments described above, a fluorescence image is captured by the first imaging system, but the invention is not limited to this and an image based on projection of special light onto an examination area and light absorption properties of the examination area may be captured.

Further, in the aforementioned embodiments, the description has been made of a case in which the light source apparatus of the present invention is applied to a rigid endoscope system, but the application of the present invention is not limited to rigid endoscope systems and it may also be applied, for example, to a flexible endoscope system.

**Claims**

1. A light source apparatus, comprising:

   a first light source (52) for emitting first light having a first wavelength range and is inputted to a light guide section (LG) that guides light to an examination area and projects the light onto the examination area;
   a second light source (50) for emitting a second light having a second wavelength range different from the first wavelength range and is inputted to the light guide section (LG); and
   an exit angle changing section (57) for simultaneously changing exit angles of the first and second light from the light guide section (LG), the first and second light being guided by the light guide section (LG) and projected onto the examination area.

2. The light source apparatus of claim 1, **characterized in that** the exit angle changing section (57) is a section that includes an incident angle changing optical system for changing the exit angles by changing incident angles of the first and second light on a light incident end face of the light guide section.

3. The light source apparatus of claim 2, **characterized in that** the exit angle changing section (57) is a section that changes the incident angles by changing a focal length of the incident angle changing optical system.

4. The light source apparatus of claim 2 or 3, **characterized in that**:

   the exit angle changing section (57) includes an upstream optical system (58) for receiving either one of the first and second light and inputting the received either one of the light to the incident angle changing optical system; and
   a ratio of the exit angles of the first and second light from the light guide section (LG) is maintained constant by changing an incident height of the either one of the light to be inputted to the incident angle changing optical system (57) by the upstream optical system (58) along with the change in the exit angles by the incident angle changing optical system (57).

5. The light source apparatus of any of claims 2 to 4, **characterized in that**:

the second light emitted from the second light source (50) is guided parallel to optical axis of the incident angle changing optical system (57) while the first light emitted from the first light source is guided perpendicular to the optical axis of the incident angle changing optical system (57); and

the apparatus includes a mirror (56) for combining the parallel-guided second light and the perpendicular-guided first light.

6. The light source apparatus of any of claims 2 to 4, **characterized in that**:

the first light emitted from the first light source is guided parallel to an optical axis of the incident angle changing optical system while the second light emitted from the second light source is guided perpendicular to the optical axis of the incident angle changing optical system; and

the apparatus includes a mirror (59) for combining the parallel-guided first light and the perpendicular-guided second light.

7. The light source apparatus of claim 2, **characterized in that** the incident angle changing optical system (57) is a zoom lens.

8. The light source apparatus of claim 4, **characterized in that** the upstream optical system (58) is a zoom beam expander.

9. The light source apparatus of claim 1, **characterized in that** the exit angle changing section includes a first light exit angle changing section (74) for changing the exit angle of the first light and a second light exit angle changing section (71) for changing the exit angle of the second light.

10. The light source apparatus of claim 9, **characterized in that** the first light exit angle changing section (74) changes the exit angle of the first light by changing an incident angle of optical axis of the first light with respect to optical axis of the light guide section (LG).

11. The light source apparatus of any of claims 1 to 10, **characterized in that** the first light source (52) is a semiconductor light source.

12. The light source apparatus of any of claims 1 to 11, **characterized in that** the first light source (52) emits near infrared light as the first light.

13. The light source apparatus of any of claims 1 to 12, **characterized in that** the second light source (50) is a visible light source.

**FIG.1**

# FIG.2

# FIG.3

FIG.4

EP 2 529 659 A1

**FIG.5**

# FIG.6

**RELATIONSHIP BETWEEN INCIDENT ANGLES OF WHITE LIGHT AND EXCITATION LIGHT**

**FIG.7**

**FIG.8**

EP 2 529 659 A1

# FIG.9

RELATIONSHIP BETWEEN INCIDENT ANGLE
AND INCIDENT HEIGHT OF WHITE LIGHT

# FIG.10

RELATIONSHIP BETWEEN FOCAL LENGTH
OF ZOOM LENS AND MAGNIFICATION RATIO
OF ZOOM EXPANDER

# FIG.11

# FIG.12

# FIG.13

RELATIONSHIP BETWEEN FOCAL LENGTH
OF ZOOM LENS AND FOCAL LENGTH
OF ZOOM LENS IN ZOOM BEAM EXPANDER

**FIG.14**

**FIG.15**

# FIG.16

RELATIONSHIP BETWEEN INCIDENT ANGLE
ON FIBER AND ILLUMINANCE DISTRIBUTION
(AT DISTANCE 50 mm)

**FIG.17**

**FIG.18**

**FIG.19**

FIG.20

FIG.21

Europäisches
Patentamt

European
Patent Office

Office européen
des brevets

## EUROPEAN SEARCH REPORT

Application Number

EP 12 17 0060

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JP 2006 204341 A (PENTAX CORP) 10 August 2006 (2006-08-10) * figure 2 * ----- | 1-13 | INV. A61B1/06 |
| Y,D | US 2002/026099 A1 (ADACHI RENSUKE [JP] ET AL) 28 February 2002 (2002-02-28) * paragraphs [0036], [0037], [0048], [0050]; figures 1,2 * ----- | 1-13 | |
| Y | US 5 143 435 A (KIKUCHI AKIRA [JP]) 1 September 1992 (1992-09-01) * column 2, lines 49-60; figures 4A,4B * ----- | 1-13 | |
| A | JP 8 094942 A (OLYMPUS OPTICAL CO) 12 April 1996 (1996-04-12) * the whole document * ----- | 1-13 | |
| A | JP 2 152103 A (FUJI PHOTO OPTICAL CO LTD) 12 June 1990 (1990-06-12) * the whole document * ----- | 1-13 | TECHNICAL FIELDS SEARCHED (IPC) A61B G02B |

The present search report has been drawn up for all claims

| Place of search | Date of completion of the search | Examiner |
|---|---|---|
| The Hague | 14 September 2012 | Lommel, André |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**                   EP 12 17 0060

This annex lists the patent family members relating to the patent documents cited in the above-mentioned European search report.
The members are as contained in the European Patent Office EDP file on
The European Patent Office is in no way liable for these particulars which are merely given for the purpose of information.

14-09-2012

| Patent document cited in search report | | Publication date | Patent family member(s) | | Publication date |
|---|---|---|---|---|---|
| JP 2006204341 | A | 10-08-2006 | NONE | | |
| US 2002026099 | A1 | 28-02-2002 | DE<br>US | 10141559 A1<br>2002026099 A1 | 07-03-2002<br>28-02-2002 |
| US 5143435 | A | 01-09-1992 | JP<br>US | 3118509 A<br>5143435 A | 21-05-1991<br>01-09-1992 |
| JP 8094942 | A | 12-04-1996 | NONE | | |
| JP 2152103 | A | 12-06-1990 | JP<br>JP | 2152103 A<br>2629323 B2 | 12-06-1990<br>09-07-1997 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20020026099 A